# EUROPEAN PATENT APPLICATION

(11) **EP 3 494 911 A1**
(43) Date of publication of application: **12.06.2019**
(21) Application number: 18215632.3
(22) Date of filing: 14.03.2014
(51) Int. Cl.: A61B 17/62, A61B 17/64

(54) **DYNAMIC FOOTPLATE**

(30) Priority: 14.03.2013 US 201361782286 P; 06.12.2013 US 201314099177
(62) Divisional of application: 14770954.7
(71) Applicant: MDPO, LLC, Sunrise, FL 33323 (US)
(72) Inventor: MARIN, Luis, E., Hileah, FL 33018 (US)
(74) Representative: Schmidbauer, Andreas Konrad

(57) **Abstract**

A dynamic foot plate assembly structured for therapeutic use adjacent the ankle area of the body comprising a base element, at least one side element extending along the ankle area, and at least one joint movably and adjustably connecting the base element to the side element for variable displacement of the base element and side element into different operative orientations. The dynamic foot plate assembly may also comprise a plurality of strut members disposed in an interconnecting relationship between either a support member and a side element, or the support member and the base element. The strut members, if present, facilitate the variable relative displacement of the base element, side element and support element into different operative orientations.

## Description

### BACKGROUND OF THE INVENTION

### Claim of Priority

The present application is based on a claim of priority under 3b U.S.C. § 119(e) to a provisional patent application filed with the U.S. Patent Office on March 14, 2013, and assigned Ser. No. 61/782286.

### Field of the Invention

This invention is directed to a support assembly for use in operative placement relative to and treatment of the ankle area including the ankle joint, foot and correspondingly disposed lower leg bones. The assembly allows for a variable orientation of at least one of its members, at least one of which is structured for the disposition of at least one transfixion pin for the engagement and treatment of a patient's ankle area.

### DESCRIPTION OF THE RELATED ART

In the medical treatment of pathologies including, but not limited to, injuries, fractures, etc. to the bone and joints, external fixator assemblies are commonly used to maintain segments of the bone in an intended and/or required stabilized orientation. By way of example, fixator assemblies of the type described may be utilized to treat the fusion of bone tissue as well soft tissue injuries, and situations involving a union of bones which otherwise are difficult to heal. As such, known or conventional fixator assemblies vary in structure, dimension and configuration and are correspondingly adapted to be used with various portions of the body to which they are attached.

Typical fixator structures include at least one connecting bars or rods as well a plurality of clamps for adjustably securing fixation pins, wires, etc. to the bone portions being affected. Further, transfixion pins or wires of the types commonly utilized may extend completely through the bony tissue or may be anchored therein, such as when the long bones of the leg are involved directly or indirectly with the treatment or healing procedure. Further, the term "transfixion member" is generally recognized in the medical field as including the describing of elongated pins which extend completely or at least partially through the bony tissue involved. In contrast, smaller, thicker "half pins" may be utilized in substantially the same manner to stabilize affected tissue but being of a length insufficient to extend completely through the affected bone, joint, etc. This term may also be used in a more generic sense in referring to stabilizing devices, other than pins, such as wires, reduction wires, screws, clamps, etc.

In addition, known external fixator assemblies of the type described may also include support rings which encircle a corresponding body member, wherein such rings or like support elements serve as a supportive base to facilitate proper location of the aforementioned transfixion members. Accordingly, it is commonly understood in the medical profession that fixator assemblies are used to maintain proper orientation of one or more of bones or bone segments relative to one another to facilitate healing or alignment.

However, the proper stabilization of tissue typically associated with the joint areas of a patient's body such as, but not limited to, the ankle joint as well as the wrist and other smaller bones associated with the hand involves additional considerations.

It would therefore be beneficial to implement a technology that incorporates dynamic aspects to allow for the acute and/or gradual relocation of a foot, ankle or leg deformity. With the dynamic properties of the assembly, a foot, ankle or leg soft tissue and bony pathology can be corrected. In addition, the calibration of the movable components of the assembly allows for ease of use and increased accuracy of adjustments, allowing the surgeon to correct complicated deformities.

### SUMMARY OF THE INVENTION

This invention is directed to a dynamic foot plate assembly primarily, but not exclusively, structured for placement adjacent an ankle area of the body. As referred to herein, the term "ankle area" is intended to describe the ankle joint, as well as bones and associated tissue of the foot and lower portions of the leg including the fibula and tibia. Further, in properly describing the intended position and orientation of the various preferred embodiments of the external fixator assembly of the present invention, terminology including "length of the ankle area" and/or "height of the ankle area" may be utilized synonymously. These terms are meant to refer to the general distance between the bottom of the foot and an area of the lower part of the leg above the ankle joint. Further the ankle area, as used herein, is meant to be descriptive of the bones and other tissue associated with the foot, ankle joint and lower leg which serve to facilitate the functioning of the ankle joint and intended, relative movements of the corresponding foot and leg connected to the ankle joint.

Accordingly, the dynamic foot plate assembly includes a configuration of side elements and joints connected to a base element intended to be disposed adjacent to the ankle area. The side elements are structured to support at least one transfixion pin or like transfixion member in operative engagement with the bones or other associated tissue of the ankle area. Consequently, assembly includes at least one base segment preferably, but not necessarily, having a curvilinear configuration substantially in the form of an arc and or/semi-circle operatively disposed at the medial and lateral longitudinal segments.

In addition, the assembly includes a configuration of joints and side elements attached to the base element and extending transversely from the base element and adjacent the ankle area. The joints and side elements are movably connected and structured to allow variable disposition of the side elements relative to the base element, including but not limited to rotation, raising/lowering, hinging/tilting, and varying the longitudinal spacing/telescoping of the configuration. Some joints may further capable of being locked or fixed, allowing for the configuration of joints and side elements to become fixed relative to one another. Joints can subsequently be unlocked, restoring the ability for the configuration to once again be articulated.

Further, at least one strut member, which may work in concert with at least one joint, extends from a support member, disposed adjacent the ankle and above the base element, and can be connected to either a base element or a side element to allow for the relative disposition of the dynamic foot plate array into a desired orientation for treatment.

One embodiment of the invention comprises a base element as previously described movably interconnected to two joints, each disposed on an opposing side of the ankle, which are in turn movably interconnected to a pair of side elements extending transversely along opposing sides of the ankle. A pair of strut members are structured to movably interconnect the base element to a support member disposed adjacent to the ankle. A second pair of strut members are structured to movably interconnect the support member to the aforementioned side elements. The four strut members and two joints are structured to cooperatively dispose the base element, side elements and support member into a desired orientation for treatment of the ankle and related areas of the lower leg.

These and other objects, features and advantages of the present invention will become clearer when the drawings as well as the detailed description are taken into consideration.

### BRIEF DESCRIPTION OF THE DRAWINGS

For a fuller understanding of the nature of the present invention, reference should be had to the following detailed description taken in connection with the accompanying drawings in which:
Figure 1 is a side view in partial cutaway of the preferred embodiment of the present invention.
Figure 2 is a side view in partial cutaway of the joint of the preferred embodiment of Figure 1.
Figure 3 is a side view in partial cutaway of the joint of the preferred embodiment of Figure 1.
Figure 4 is a side view in partial cutaway of the joint of the preferred embodiment of Figure 1.
Figure 5 is a side view in partial cutaway of the joint of the preferred embodiment of Figure 1.
Figure 6 is a side view in partial cutaway of a plurality of strut members of the preferred embodiment of Figure 1.
Figure 7 is a side view in partial cutaway of one of a plurality of strut members as disposed in the preferred embodiment of Figure 1.
Figure 8 is a front view in partial cutaway of one of a plurality of strut members, with thin lines used for clarity to contrast the depiction of the internal structure of the strut member.
Figure 9 is a front view of another embodiment of the present invention when operatively positioned relative to an ankle area of a patient.
Figure 10 is a side view of the embodiment of Figure 9 when operatively positioned relative to an ankle area of a patient.
Figure 11 is a side view of the embodiment of Figure 9.
Figure 12 is a view in partial cutaway of one of a plurality of strut members as structured in another embodiment of the present invention.
Figure 13 is a view in partial cutaway of one of a plurality of joints as structured in an embodiment of the present invention.

Like reference numerals refer to like parts throughout the several views of the drawings.

### DETAILED DESCRIPTION OF THE PREFERRED EMBODIMENT

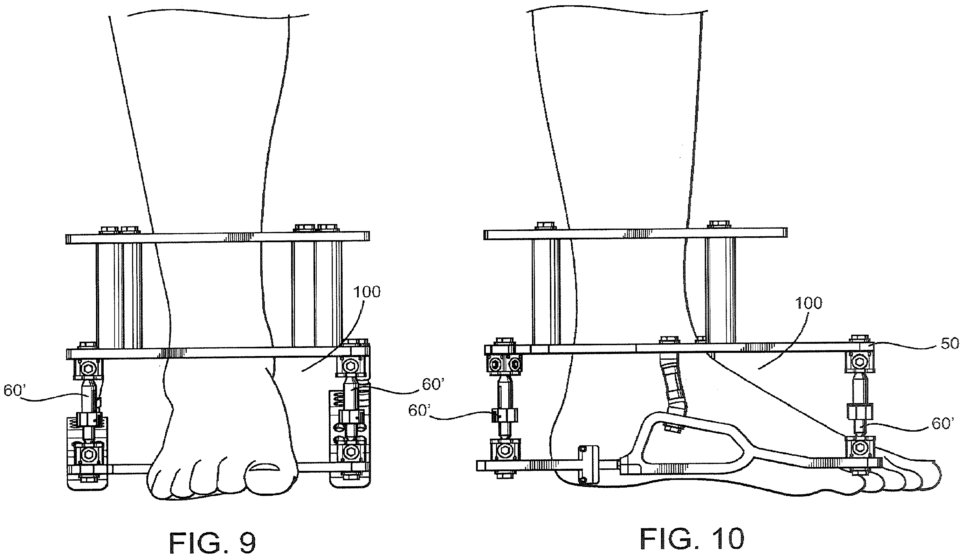

As represented in the accompanying figures, the present invention is directed to a dynamic foot plate assembly generally indicated as 1. As demonstrated the dynamic foot plate assembly 1 is structured to be operatively position and used in a location substantially adjacent the ankle area 100 of a patent as indicated in Figures 9 and 10. As set forth above, the ankle area 100 is meant to be descriptive of substantially the entire area, which includes the ankle joint, foot, corresponding portions of the leg bones, including the fibula and tibia, as well as the associated components and tissue. In addition, the terms "height" and "length" of the ankle area 100 are used synonymously herein and refer to the distance from substantially the bottom of the foot, as at, to at least a portion of the long bones of the leg, as at 100.

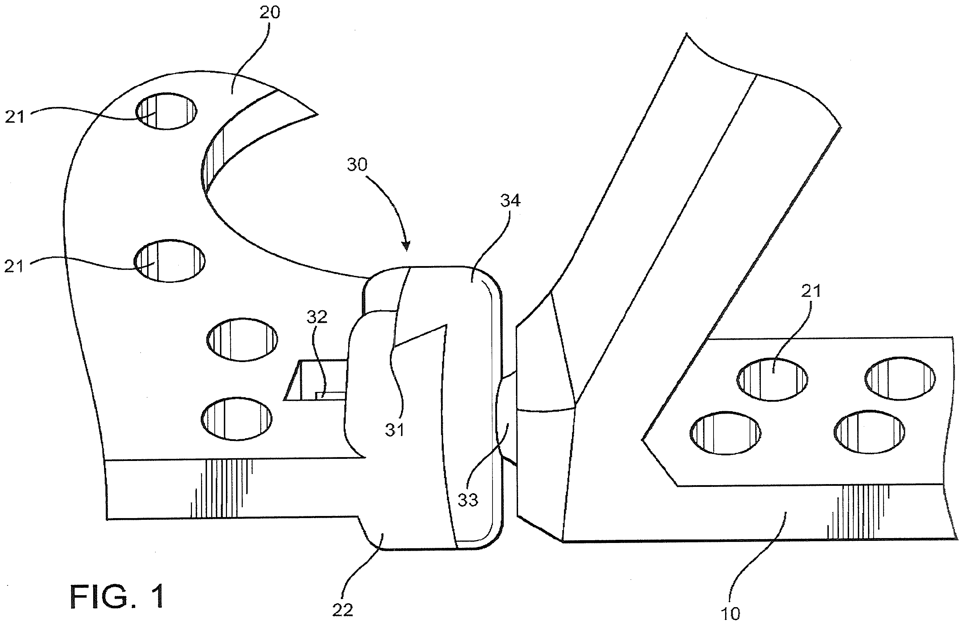

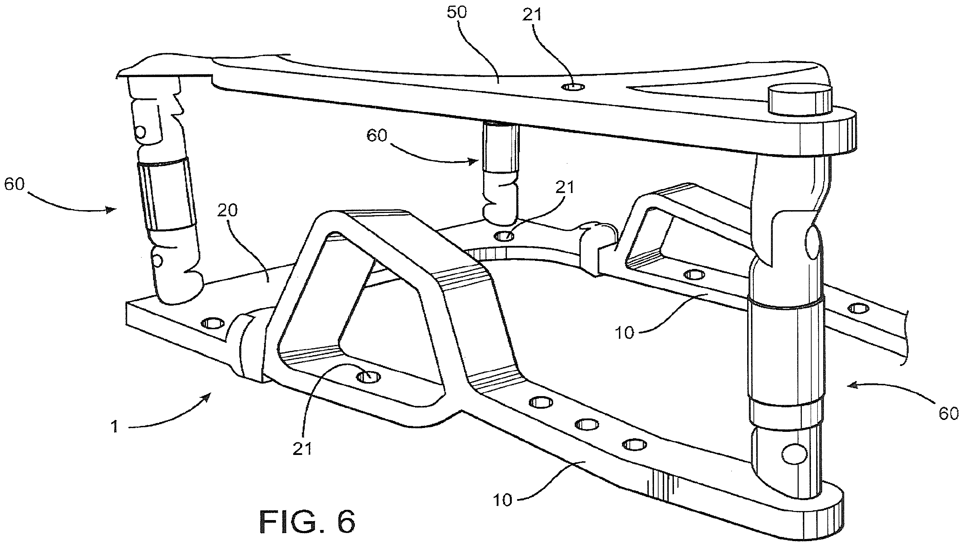

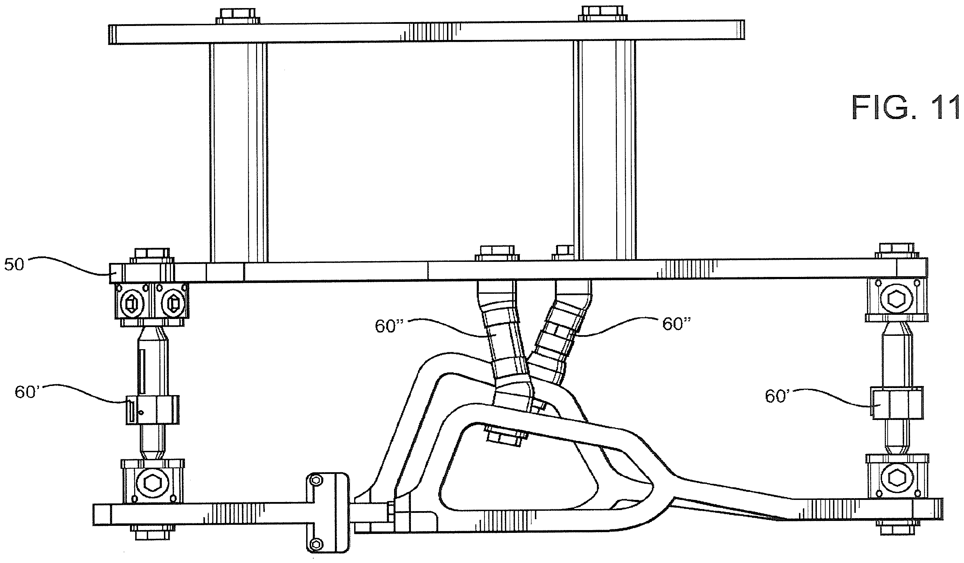

Accordingly, the dynamic foot plate assembly 1 comprises a base element generally indicated as 20 movably interconnected to at least one joint generally indicated as 30. In Figure 11, a possible alternate embodiment of a joint is given at 30'. With reference to Figure 6, additionally, the foot plate assembly 1 further comprises at least one strut member generally indicated as 60 interconnected to at least one support member generally indicated as 50 and at least one side element generally indicated as 10. With primary reference to Figure 1, the base element 20 defining at least a portion of the dynamic foot plate assembly 1 in the preferred embodiment includes a curvilinear configuration which may be more specifically defined by an arcuate or semi-circular shape, but any suitable shape will suffice. As such, the base element 20 terminates in oppositely disposed free ends 22. Further, a plurality of apertures 21 or other appropriate structure are positioned substantially along the length of the base element 20, at least one side element 10, and the support member 50, and are provided to facilitate connection of at least one fixation struts preferably using fixation bolts, which are not shown for purposes of clarity. Such struts and interconnecting fixation bolts are used to support and/or dispose the base segment 20 in a stabilized position relative to the ankle area 100. The opposite ends of such struts, to which the base segment 20 is connected, may be secured to a halo-type ring located above the ankle area 100 along the length of the leg and in surrounding relations to the bones of the leg. Such anchoring of the halo ring provides stabilizing support to the base element 20 by virtue of the interconnection between the halo ring and the base element 20 by the plurality of strut members.

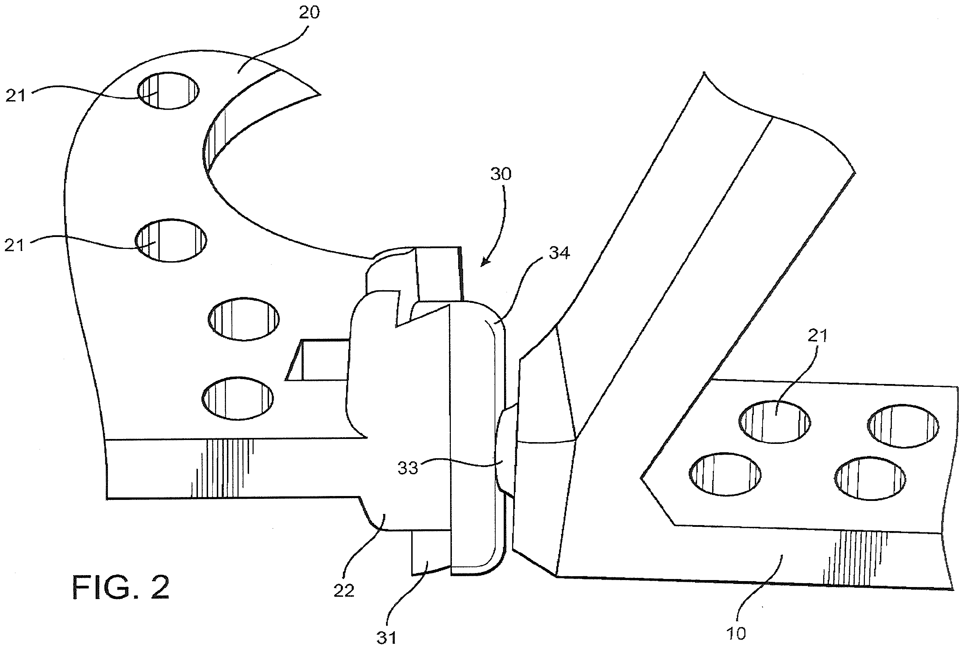

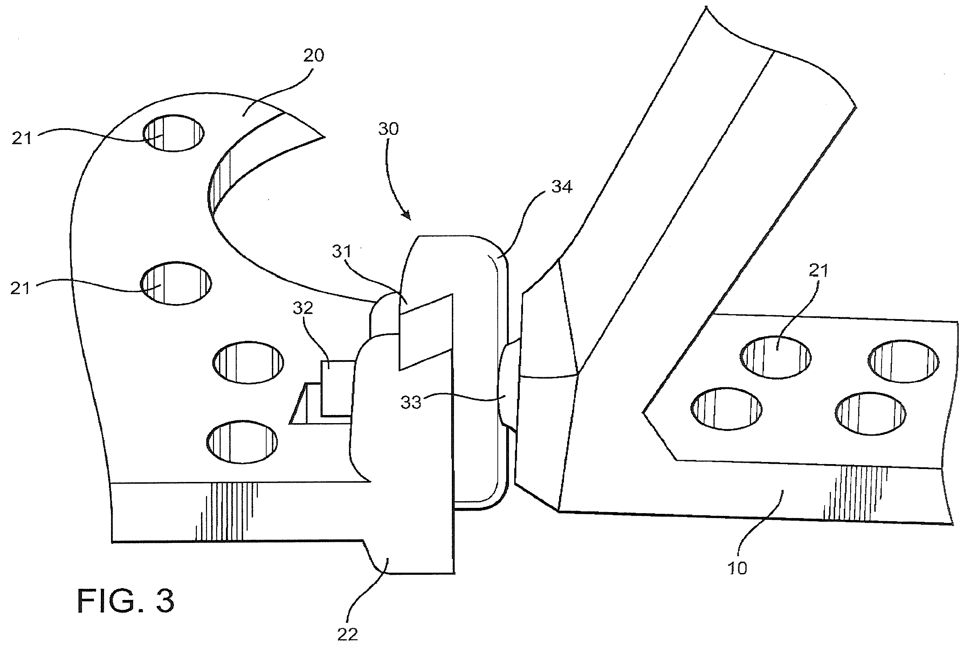

With primary reference to Figure 1, the joint 30 as depicted in the preferred embodiment comprises a joint housing 34, an extension element 32, and a pivot element 33. The joint housing 34 can be made of any sufficiently rigid or sturdy material, such as and in the depicted embodiment is centrally apertured to receive the extension element 32, which extends substantially into and, in this case, through the joint housing 34. In other embodiments, the extension element 32 may only partially recess into the joint housing. The joint housing 34 in the preferred embodiment is curvilinear about its circumference, but any suitable geometric configuration will suffice. The joint housing 34 also features a set of flanges 31 extending linearly in a direction substantially perpendicular to the axis of the joint housing 34. The flanges 31 facilitate a confronting engagement with the adjoining structure which, in the depicted embodiment is the base element 20, but in alternative embodiments may be another part of the dynamic foot plate assembly 1 such as a side element 10 properly configured for similar confronting engagement of the flanges 31. The flanges 31 maintain interconnection between the joint housing 34 and the base element 20 while simultaneously facilitating linear movement substantially resembling sliding in the direction of the extension of the flanges 31 as depicted in Figures 2 and 3. This sliding, or vertical displacement, confers a significant benefit to a medical professional using the dynamic foot plate assembly 1 by allowing the adjustment into a desired orientation by varying the disposition of the base element 20 relative to at least one side element 10 both prior to the onset of and during treatment.

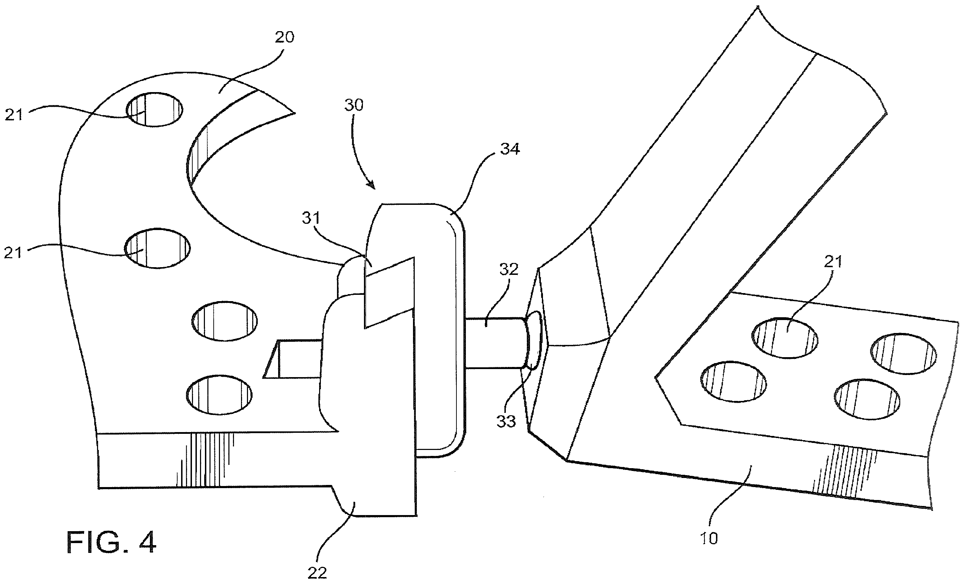

The extension element 32 may be a longitudinal member that extends wholly or substantially through the aperture of the joint housing 34. The extension element 32 is coaxially aligned with the aperture in the joint housing 34. The extension element 32 may resemble a screw, bolt or other threaded rod-like structure capable of extension through or partially through the aperture of the joint housing 34. The extension element 32 in the preferred embodiment is a threaded longitudinal member, with the threads extending substantially along the outer length of the extension element 32 and facilitating a frictional confronting engagement with opposing threads lining the interior of the central aperture of the joint housing 34. The structure of the extension element 32 allows for the variable disposition or displacement of the base element 20 and at least one side element 10, or alternatively between two side elements 10, directed along the axis of the joint housing 34. This is depicted in Figure 4. Variable disposition is achieved by rotation of the extension element 32 about its axis, which can either extend or retract the extension element 32 through the aperture of the joint housing 34 via the utilization of the threads extending substantially along the length of the extension element 32.

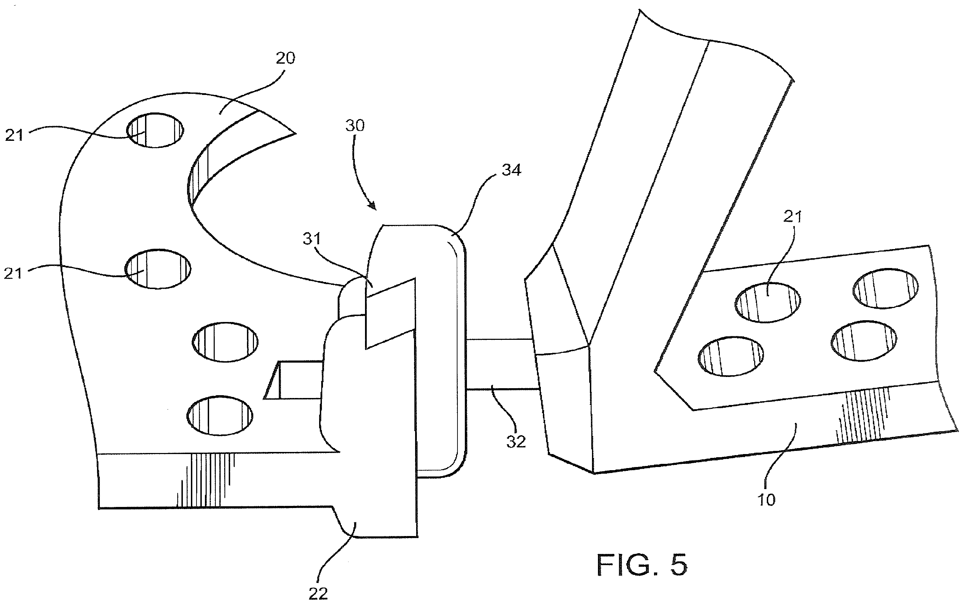

Attached to the extension element 32 or, alternatively, one end of the extension element 32 itself, is a pivot element 33 structured for an at least partially universal range of motion. The pivot element 33 may substantially resemble a ball in socket. The pivot element 33 is The pivot element 33 facilitates a tilting motion defined as the variance of the angular disposition of the axis of the side element 10 relative to the base element 10 as depicted in Figures 4 and 5. Alternatively, in another embodiment, the joint 30 could be configured to connect two side elements 10, allowing for a similar tilting motion by way of the pivot element 33, disposed in a socket in one of the two side elements 10, to vary the angular disposition of the axes between the two side elements 10. The pivot element 33 also facilitates the relative varying of the disposition of a base element 20 and a side element 10, as shown in the preferred embodiment, or between two side elements 10, in a lateral direction toward or away from the ankle. Finally, the joint 30 may also facilitate a rotational or rotary movement in such a way that does not vary the angular disposition of the base element 20 and side element 10, or as between two side elements 10. It is important to note that none of these movements is exclusive of any other, and indeed the varying of relative disposition of a side element 10 with either a base element 20 or another side element 10 facilitated by the pivot element 33 can be a compound movement that consist of at least one of the aforementioned motions, tilting, lateral or rotary, necessary for a medical professional or other operator to properly dispose a side element 10 into a predetermined orientation to effect treatment.

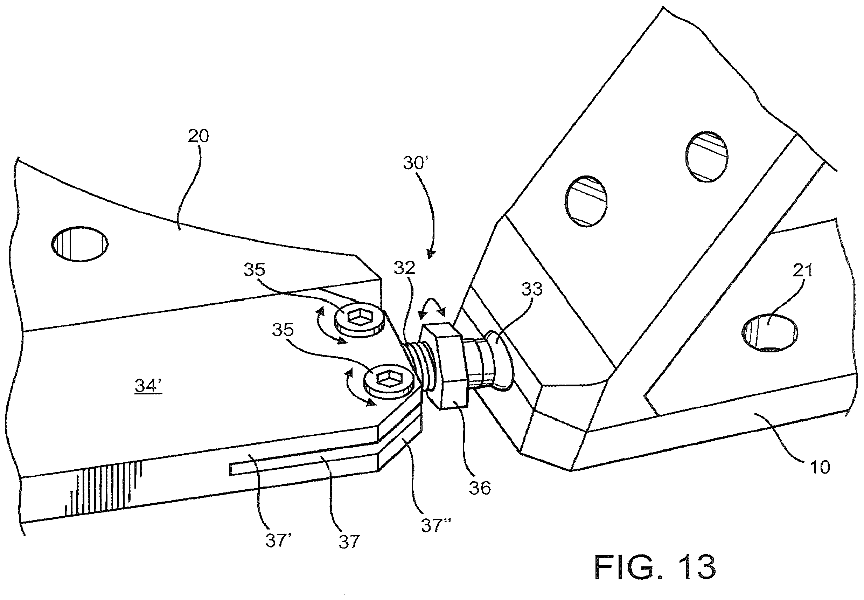

Another embodiment of the joint is given at 30' as shown in Figure 13. This embodiment may further comprise a nut 36 or similar centrally apertured structure disposed upon the extension element 32 and coaxially aligned therewith. The nut 36 is capable of translation along the extension element 32 and can be caused to be placed in confronting engagement with the side element 10. This confronting engagement between the nut 36 and the side element 10 restricts or eliminates the movement of the side element 10 facilitated by the pivot element 33 as described above. Additionally, the joint 30' may comprise an alternate embodiment of a joint housing 34' interconnected to a base element 20 or side element 10. This joint housing 34' may comprise a plurality of bolts, nuts, or other compression elements, given as 35. These compression elements 35 may be threaded such that a rotational force, such as with a screwdriver, hex key, wrench, etc. is applied about the central axis, the head of any one of the compression elements 35 exerts a compressive force upon the joint housing 34'. The result of the compressive force is to increase the frictional forces exerted upon the extension element 32, causing the extension element 32 to become frictionally locked in a desired orientation. Consequently, the joint housing 34' may be structured in such a way that the frictional force component of the frictional confronting engagement, as previously described, exerted upon the extension element 32 by the joint housing 34' is capable of being varied. One possible way of doing that is by the inclusion of a gap 37 or similar spacing between two separate parts 37', 37" of the joint housing 34', in which the extension element 32 is disposed. As such, a compressive force exerted by a compression element 35, through e.g. a rotation of the compression element 35 as described above, causes the gap 37 to decrease in width, resulting in the substantially fixed "clamping" of the extension element 32 there between. Consequently, the two parts 37', 37" of the joint housing 34' are forced together, and in turn increase the compression and thus frictional forces, i.e. clamping forces, exerted upon the extension element 32. Thus, the extension element 32 is sandwiched between the two parts 37', 37", causing the extension element 32 to become frictionally locked or clamped in a desired orientation. Rotating the compression element or elements 35 in the opposite direction causes the gap 37 to widen, decreasing the aforementioned clamping forces and unlocking the compression element 32, restoring its capability for previously described movement.

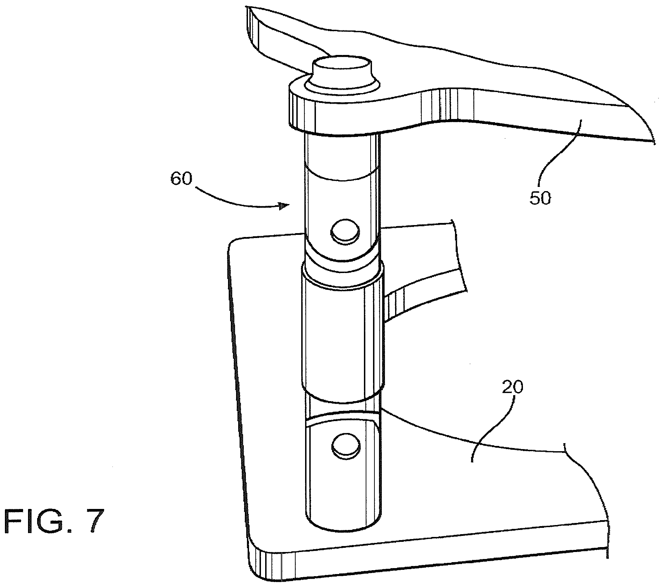

Additionally, disposed above the base element 20 and at least partially surrounding the ankle is a support member 50, which is depicted in Figures 6 and 7. With primary reference to Figure 6, the support member 50 has substantially along its length a plurality of apertures for the connection of fixation struts, preferably using fixation bolts, which are known to those practiced in the art and are used to effect treatment of the ankle or lower leg. The support member 50 is preferably curvilinear, with a cross section reminiscent of an ellipse, though any other shape that facilitates disposition that at least partially surrounds the ankle is suitable. At least one strut aperture 51 is present on the support member 50 and extends partway or totally through the support member 50 and allows for attachment of the strut member 60 to the support member 50. The method of attachment of the preferred embodiment and alternatives will be discussed in detail below.

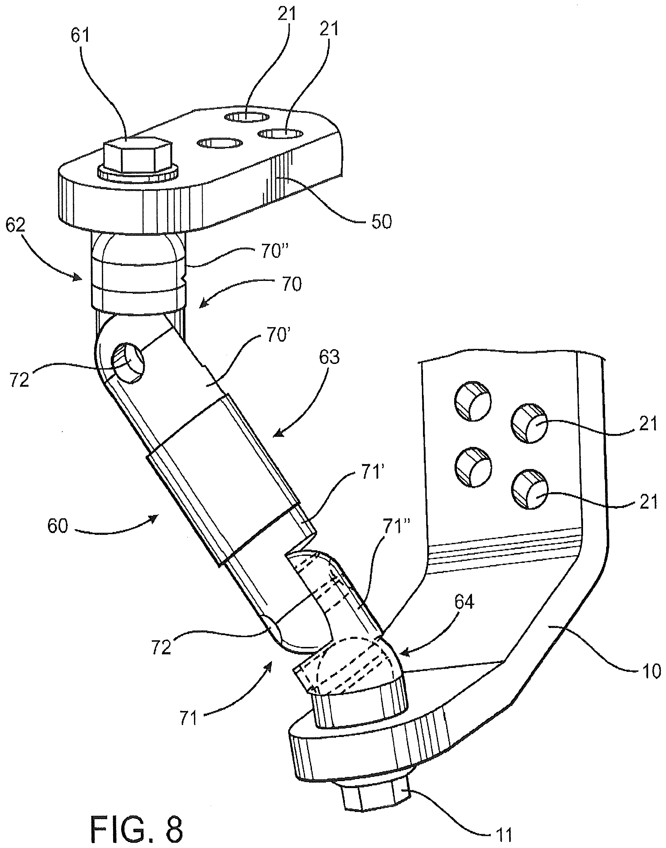

With primary reference to Figure 8, a strut member 60 comprises a pair of strut attachment elements 61 that attach one end of the strut member 60 to a support member 50 and the opposing end to the side element 10 or, as shown in Figure 7, a base element 20. With reference to Figures 9, 10, and 11, possible alternate embodiments of strut members are given at 60' and 60". Returning to Figure 8, the strut attachment element 61 can be any means of fixed attachment that allows for confronting engagement between the strut member 60 and the desired attaching element, be it the aforementioned side element 10, base element 20, or support member 50 such as a threaded bolt or suitably strong adhesive substance. As depicted in the preferred embodiment, the strut attachment element is comprised of a nut that fastens a threaded bolt that passes through an aperture in the desired attaching element to ensure abutment between the attaching element and the strut member 60. The strut member 60 is structured so as to facilitate the variable disposition of the support member 50, a base element 20, and/or a side element 10 relative to the ankle. The strut attachment element 61 that passes through the support member 50 is attached to a first strut member 62 and abuts the support member 50. The first housing 62 can be socketed or otherwise structured to receive one end of the first hinge 70, the structure of which will be discussed in detail below. On the opposite end of the first hinge 70 is a second housing 63. The second housing 63 is socketed at either end, or can be centrally apertured, and is structured to receive one end of the first hinge 70 and one end of the second hinge 71 as depicted in Figure 8. The end of the second hinge 71 is disposed within a third housing, which abuts either a base element 20 or a side element 10 in a confronting engagement facilitated by the second of two strut attachment elements 61.

The first hinge 70 is comprised of a primary first hinge member 70', a secondary first hinge member 70", and a hinge fastener 72. The secondary first hinge member 70" is disposed with a hollow, socket or other similar recess in the first housing 62 in such a way as to facilitate the rotary motion of the secondary first hinge member 70" about its central axis. The exposed end of the secondary first hinge member 70" is apertured to receive a hinge fastener 72. Abutting the secondary first hinge member 70" is the primary first hinge member 70', which is similarly apertured as shown in Figure 8 to receive the hinge fastener 72. The abutting ends of the primary and secondary first hinge members 70' and 70" are cooperatively structured and configured to pivot about a common axis.

Additionally, one of a pair of hinge fasteners 72 joins the primary first hinge member 70' and the secondary first hinge member 70" and facilitates their rotational movement about an axis defined by the central axis of the hinge fastener 72. The hinge fastener 72 can be a bolt and nut or any similar fastening structural composition that allows for tightening to adjust the confrontation between the primary first hinge member 70' and secondary first hinge member 70". By adjusting the confrontation, it is possible to cause the first hinge 70 to become frictionally locked, which is desirable when disposing the dynamic foot plate array 1 into a predetermined position for treatment. When the first hinge 70 is frictionally locked, reducing the tensile forces directed along the central axis of the hinge fastener 72 will restore the ability for the primary first hinge member 70' and secondary joint hinge member 70" to rotate about the aforementioned axis. The primary second hinge member 71' and the secondary second hinge member 72" are similarly attached with the second of a pair of hinge fasteners 72, the function of which is substantially the same as set forth above.

Furthermore, a second housing 63, which may be socketed on each end or else centrally apertured, is structured to receive in one end the primary first hinge member 70' and in the other end the primary second hinge member 71', as shown in Figure 8. The second housing 63 is structured to interconnect the primary members 70' and 71'. The second housing 63 is further structured to facilitate the rotational movement of the primary members 70' and 71' about an axis, defined as the central axis of the second housing 63, independent of the other primary member.

The second hinge 71 comprises the primary second hinge member 71' and a secondary second hinge member 71" cooperatively structured and configured to pivot about a common axis, defined as the central axis of the aforementioned hinge fastener 72 that joins the two members 71' and 71".

A third housing 64 is pivotally interconnected to the secondary second hinge member 71", is structured to facilitate an at least partially universal range of motion of the secondary second hinge member 71", and may substantially resemble of that of a ball in socket.

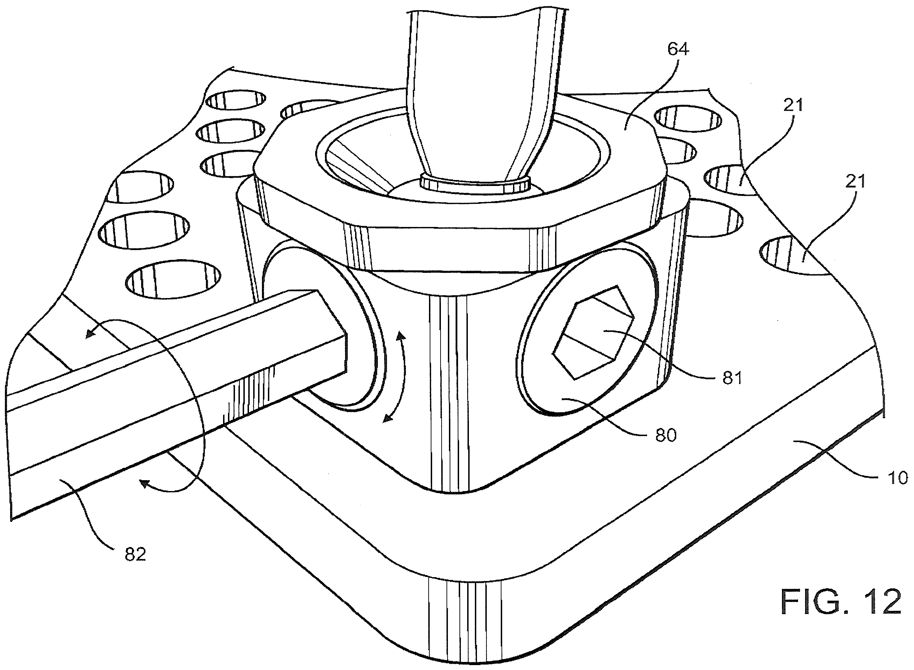

Another embodiment of the present invention is shown in Figure 12 and features a locking mechanism providing for the disposition of the strut member 60 into fixed orientation. The third housing 64 comprises at least one, but may comprise a plurality of, apertures structured to receive a locking bolt 80. The locking bolt 80 is coaxially aligned with the aperture of the third housing 64. The aperture in the third housing 64 is in abutting confrontation with the locking bolt 80, and such abutting confrontation is further defined by the complementary threading of the confronting surfaces of the locking bolt 80 and the third housing 64 as is common of a bolt and a nut. As a result, a rotary force upon the locking bolt 80 about its central axis, which as discussed above is aligned with the central axis of the respective aperture of the third housing 64 into which the locking bolt 80 is inserted, causes the locking bolt 80 to translate along the central axis. Consequently, a rotary force, when applied to the locking bolt 80, can be made to cause the locking bolt 80 to press against the ball of the aforementioned ball in socket assembly, and thereby apply a frictional force sufficient to cause the ball to become frictionally locked and thus unable to move within the socket. The locking bolt 80 may itself be apertured, the aperture 81 being structured to receive a "tool" 82, defined as a tension member structured to provide the rotary force above described. In other embodiments, the locking bolt 80 may be structured to accommodate alternative types of tools 82, such as a Phillips screwdriver, flathead screwdriver, hex key, socket wrench, etc., to facilitate the rotary operation of the locking bolt 80.

Since many modifications, variations and changes in detail, can be made to the described preferred embodiment of the invention, it is intended that all matters in the foregoing description and shown in the accompanying drawings be interpreted as illustrative and not in a limiting sense. Thus, the scope of the invention should be determined by the appended claims and their legal equivalents.

Now that the invention has been described,

### FURTHER SUMMARY OF THE INVENTION

1. A dynamic footplate assembly structured for therapeutic use adjacent the ankle area of the body, said footplate assembly comprising:
   a base element operatively disposed adjacent a posterior portion of the ankle area,
   at least one side element disposed adjacent to and extending along a foot of the ankle area,
   at least one joint movably and adjustably connecting said base element to said one side element, and
   said one joint structured for variable displacement of said one side element relative to said base into different operative orientations.
2. A dynamic footplate assembly as recited in 1 wherein said different operative orientations comprise a variable longitudinal spacing between said base and said one side element.
3. A dynamic footplate assembly as recited in 2 wherein said different operative orientations further comprise a variable height spacing between said base and said one side element.
4. A dynamic footplate assembly as recited in 3 wherein said different operative orientations further comprise a variable angular orientation of said one side element relative to said base.
5. A dynamic footplate assembly as recited in 1 wherein said different operative orientations further comprise a variable height spacing between said base and said one side element.
6. A dynamic footplate assembly as recited in 1 wherein said different operative orientations further comprise a variable angular orientation of said one side element relative to said base.
7. A dynamic footplate assembly as recited in 1 further comprising at least two side elements each disposed adjacent to and extending along a different side of the foot; at least two joints each movably and adjustably connecting said base to a different one of said side elements; each of said joints structured for variable displacement of said a corresponding side element relative to said base into different operative orientations.
8. A dynamic footplate assembly as recited in 7 wherein said different operative orientations comprise a variable longitudinal spacing between said base and corresponding ones of said side elements.
9. A dynamic footplate assembly as recited in 8 wherein said different operative orientations further comprise a variable height spacing between said base and corresponding ones of said side elements.
10. A dynamic footplate assembly as recited in 9 wherein said different operative orientations further comprise a variable angular orientation of corresponding ones of said side elements relative to said base.
11. A dynamic footplate assembly as recited in 7 wherein said different operative orientations further comprise a variable height spacing between said base and corresponding ones of said side elements.
12. A dynamic footplate assembly as recited in 7 wherein said different operative orientations further comprise a variable angular orientation of corresponding ones of said side elements relative to said base.
13. A dynamic footplate assembly as recited in 1 wherein said one joint comprises a housing reciprocally positionable with said one side element in a direction substantially transverse to said base.
14. A dynamic footplate assembly as recited in 13 wherein said one joint comprises an extension element reciprocally positionable with said one side element in a direction substantially parallel to a plane of said base.
15. A dynamic footplate assembly as recited in 14 wherein said one joint comprises a pivot structure rotationally interconnecting said one side element to said base.
16. A dynamic footplate assembly as recited in 15 wherein said pivot structure comprises a ball and socket assembly disposed to on both said one joint and said one side element and structured for at least partial universal movement of said one side element relative to said base.
17. A dynamic footplate assembly as recited in 1 further comprising a support member disposed along a length of the ankle area in space relation to said base element and one side element; at least one strut member movably interconnecting said one side element to said support member.
18. A dynamic footplate assembly as recited in 17 wherein said one strut member is structured to facilitate a variable angular orientation of said one side element relative to said support member.
19. A dynamic footplate assembly as recited in 18 wherein said one strut member is structured to facilitate a variable angular orientation of said one side element relative to said base element.
20. A dynamic footplate assembly as recited in 18 wherein said one strut member is pivotally connected to said one side element and said support member.
21. A dynamic footplate assembly as recited in 20 wherein said one strut member is rotationally connected to said one side element and said support member.
22. A dynamic footplate assembly as recited in 17 wherein said one strut member comprises a first hinge member, said first hinge member comprising a primary first hinge member and a secondary first hinge member, said primary first hinge member pivotally interconnected to said secondary first hinge member, said first hinge member rotationally interconnected to said support member.
23. A dynamic footplate assembly as recited in 22 wherein said one strut member further comprises a strut housing and a second hinge member, said housing rotationally interconnecting said second hinge member to said first hinge member, said second hinge member comprising a primary second hinge member and a secondary second hinge member, said primary second hinge member pivotally interconnected to said secondary second hinge member.
24. A dynamic footplate assembly as recited in 23 wherein said second hinge member comprises a pivot structure rotationally interconnecting said second hinge member to said base.
25. A dynamic footplate assembly as recited in 24 wherein said pivot structure comprises a ball and socket assembly disposed on both said second hinge and said base and structured for at least partial universal movement of said base relative to said support member.
26. A dynamic footplate assembly as recited in 23 wherein said second hinge member comprises a pivot structure rotationally interconnecting said second hinge member to said side element.
27. A dynamic footplate assembly as recited in 26 wherein said pivot structure comprises a ball and socket assembly disposed on both said second hinge and said base and structured for at least partial universal movement of said side element relative to said support member.
28. A dynamic footplate assembly as recited in 17 wherein said one strut member is structured to selectively dispose said base element and said support member into fixed relation with one another.
29. A dynamic footplate assembly as recited in 23 wherein said one joint is structured to selectively dispose
   said base element and said one side element into fixed relation with one another.
30. A dynamic footplate assembly structured for therapeutic use adjacent the ankle area of the body, said footplate assembly comprising:
   a base element;
   two side elements;
   two joints each disposed in an interconnecting relation between said base element and a different corresponding one of said side elements;
   a plurality of strut members;
   at least a first two of said plurality of strut members being disposed in an interconnecting relation between said base element and a support member;
   at least a second two of said plurality of strut members each being disposed in an interconnecting relation between said support member and a different corresponding one of said side elements;
   each of said joints structured for variable displacement of corresponding ones of said side elements relative to said base element;
   each of said strut members structured to facilitate a variable angular orientation of corresponding ones of said side elements relative to both said support member and said base element.
31. A dynamic footplate assembly as recited in 30 wherein said variable displacement comprises a variable angular orientation between said base element and said side elements.
32. A dynamic footplate assembly as recited in 31 wherein said variable displacement further comprises a variable longitudinal spacing between said base element and said side elements.
33. A dynamic footplate assembly as recited in 32 wherein said variable displacement further comprises a variable height spacing between said base element and said side elements.
34. A dynamic footplate assembly as recited in 30 wherein at least one of said plurality of strut members is pivotally connected to at least one of said side elements and said support member; and wherein at least one of said plurality of strut members is pivotally connected to said base element and said support member.
35. A dynamic footplate assembly as recited in 34 wherein at least one of said plurality of strut members is structured to facilitate a variable angular orientation of at least one of said side elements relative to said support member.
36. A dynamic footplate assembly as recited in 35 wherein at least one of said plurality of strut members is structured to facilitate a variable angular orientation of at least one of said side elements relative to said base element.

## Claims

1. A dynamic footplate assembly structured for therapeutic use adjacent the ankle area of the body, said footplate assembly comprising:
a base element;
two side elements;
two joints each disposed in an interconnecting relation between said base element and a different corresponding one of said side elements;
a plurality of strut members;
at least a first two of said plurality of strut members being disposed in an interconnecting relation between said base element and a support member;
at least a second two of said plurality of strut members each being disposed in an interconnecting relation between said support member and a different corresponding one of said side elements;
each of said joints structured for variable displacement of corresponding ones of said side elements relative to said base element;
each of said strut members structured to facilitate a variable angular orientation of corresponding ones of said side elements relative to both said support member and said base element.

2. A dynamic footplate assembly as recited in claim 1 wherein said variable displacement comprises a variable angular orientation between said base element and said side elements.

3. A dynamic footplate assembly as recited in claim 2 wherein said variable displacement further comprises a variable longitudinal spacing between said base element and said side elements.

4. A dynamic footplate assembly as recited in claim 3 wherein said variable displacement further comprises a variable height spacing between said base element and said side elements.

5. A dynamic footplate assembly as recited in claim 1 wherein at least one of said plurality of strut members is pivotally connected to at least one of said side elements and said support member; and wherein at least one of said plurality of strut members is pivotally connected to said base element and said support member.

6. A dynamic footplate assembly as recited in claim 5 wherein at least one of said plurality of strut members is structured to facilitate a variable angular orientation of at least one of said side elements relative to said support member.

7. A dynamic footplate assembly as recited in claim 6 wherein at least one of said plurality of strut members is structured to facilitate a variable angular orientation of at least one of said side elements relative to said base element.
